Europäisches Patentamt

European Patent Office

Office européen des brevets

(11) Publication number: **0 227 833**
**B1**

## EUROPEAN PATENT SPECIFICATION

(45) Date of publication of patent specification: **16.01.91**

(51) Int. Cl.⁵: **C 12 P 21/02**

(21) Application number: **85903688.1**

(22) Date of filing: **16.07.85**

(86) International application number:
**PCT/JP85/00399**

(87) International publication number:
**WO 87/00553 29.01.87 Gazette 87/03**

(54) PROCESS FOR PREPARING HETERO-PROTEIN.

(43) Date of publication of application:
**08.07.87 Bulletin 87/28**

(45) Publication of the grant of the patent:
**16.01.91 Bulletin 91/03**

(84) Designated Contracting States:
**BE CH DE FR GB LI NL SE**

(56) References cited:
**EP-A-0 145 390**
**EP-A-0 147 819**
**GB-A-2 138 842**

(73) Proprietor: **THE GREEN CROSS CORPORATION**
**3-3, Imabashi 1-chome Chuo-ku**
**Osaka-shi (JP)**

(72) Inventor: **WATANABE, Hironobu**
**4-7-23-1408, Nankonaka**
**Suminoe-ku, Osaka-shi Osaka 559 (JP)**
Inventor: **ISHIKAWA, Hideyuki**
**33-402, Senriokanaka**
**Suita-shi Osaka 565 (JP)**
Inventor: **NAGAI, Masanori**
**10-10, Nishiyamawaki**
**Yamata-shi Kyoto 614 (JP)**
Inventor: **ARIMURA, Hirofumi**
**2-18-1-401, Uenosaka**
**Toyonaka-shi Osaka 560 (JP)**

(74) Representative: **Lewin, John Harvey et al**
**ELKINGTON AND FIFE Beacon House 113**
**Kingsway**
**London WC2B 6PP (GB)**

**Description**

## TECHNICAL FIELD AND DISCLOSURE OF THE INVENTION
TECHNICAL FIELD

This invention relates to a method of producing heterologous proteins by recovering heterologous proteins from genetically engineered, heterologous protein-producing transformants.

BACKGROUND ART

The recombinant DNA technology has made it possible to cause heterologous proteins to be expressed and produced in bacteria and other hosts.

For instance, many human proteins, inclusive of all known types (α, β, γ) of human interferon (hereinafter "HIFN"), urokinase and TPA (human tissue-derived plasminogen activating factor), are produced in hosts by transforming the hosts with DNAs coding for such proteins, and growing the resultant transformants under conditions suited for the expression of said heterologous proteins. However, such heterologous proteins are often insolubilized in the hosts and precipitate therein. When a desired protein forms such a precipitate, the recovery thereof encounters the following problems. Firstly, it is necessary to separate the protein occurring as a precipitate in the host from the host substances and, furthermore, to solubilize said protein. Secondly, desired proteins are often in a biologically inactive form. Thirdly, desired proteins are reduced in molecular weight by the presence of host-derived protease in the step of purification.

Two methods have been proposed in the art to solve these problems. One method consists in the treatment with a protein-denaturing agent (Japanese Patent Application laid open under Kokai No. 59—161321) and the other in the treatment with guanidine hydrochloride (USP 4,476,049). However, these methods do not afford satisfactory recovery of desired proteins.

Guanidine hydrochloride is used in the purification of heterologous proteins in GB—A—2138842, EP—A—0147819 and EP—A—0145390. JP—A—59—169494 discloses a buffer used in protein extractions containing NaCl among other components.

DISCLOSURE OF THE INVENTION

It is an object of the invention to provide a method of recovering heterologous proteins from genetically engineered transformants in higher yields by solubilizing said heterologous proteins occurring in the form of precipitates in said transformants, and by restoring their active form as confirmable by bioassay and, more particularly, to provide a method of preventing said proteins from being reduced in molecular weight in the purification step.

The invention thus provides a method of producing heterologous proteins, by cultivating genetically engineered, heterologous protein-producing transformants to thereby cause production of said heterologous proteins, and of recovering the heterologous proteins thus produced, which comprises carrying out the treatment for recovery of said heterologous proteins from said transformants bearing the heterologous proteins produced therein, in the presence of magnesium chloride.

(a) Starting materials

The starting material to be used in the practice of the invention is a genetically engineered transformant capable of allowing expression of a heterologous protein. The host should be one which allows production and accumulation of the heterologous protein in said host, in particular, a microbe. A preferable example is *Escherichia coli*.

Such heterologous protein-producing transformant can be obtained by gene manipulation using an appropriate known method or a modification thereof. Such method is disclosed, for example, in Japanese Patent Application No. 59—37119 (Applicant: The Green Cross Corporation) for the production of urokinase, in Japanese Patent Application No. 59—229864 (Applicant: The Green Cross Corporation) for the production of IFN-γ, in Japanese Patent Applications laid open under Kokai No. 58—104887 and Kokai No. 59—48082 for the production of HBsAg, or in Japanese Patent Application laid open under Kokai No. 59—42321 for the production of TPA.

(b) Recovery of heterologous proteins from transformants

The "recovery of a heterologous protein" herein means the step of isolating the heterologous protein accumulated in the transformant mentioned above under (a) from said transformant, and purifying said protein.

The chloride ion is caused to be present in the whole recovery step or in one or more sections of said step, preferably throughout the whole step of recovery. Since, in said recovery, the transformant is first subjected to a procedure for its lysis in a buffer solution as a general rule, it is preferable that the chloride ion should be caused to be present in said buffer solution. It is particularly preferable that, thereafter, the chloride ion should not be removed until a high-level purification is completed.

The chloride ion is caused to be present in the form of a salt capable of releasing the chloride ion, i.e. magnesium chloride.

The chloride ion is preferably caused to be present in a concentration of not less than 0.1 M, preferably about 0.2—3 M, on the salt form basis.

In the following, the recovery step is described in more detail.

(b—1) Preparation of a buffer:

The buffer solution to be used in the recovery of heterologous proteins generally has a pH of 5—10, preferably a pH of 7—8. 250 mM Tris hydrochloride buffer (pH 8.0) may be mentioned as an example. It is preferable to add the chloride ion-releasing salt to the buffer solution. The final concentration of the salt added should be not less than 0.1 M, preferably 0.2—3 M.

(b—2) Extraction treatment:

The transformant is suspended in the above buffer solution (to a measured $OD_{650}$ value of 100—200) and the suspension is subjected to extraction treatment, such as Dynomill treatment, sonication or French press treatment. The Dyno-mill treatment is suitably carried out with Ø 0.1 mm gliding beads at a revolution rate of about 3,000 r.p.m. for 1—10 minutes, preferably 1—3 minutes.

(b—3) purification:

After the above extraction treatment, the heterologous protein is generally purified. For the purification, appropriate conventional techniques known for protein purification can be used. For example, the fractionation methods based on solubility differences (e.g. fractionation with ammonium sulfate, fractionation with ethanol, fractionation with acrinol) and centrifugation are suitably used.

The fractionation with ammonium sulfate is carried out by combining the step of fractionation with 10—20% saturated ammonium sulfate and the step of fractionation with 40—50% saturated ammonium sulfate.

The centrifugal separation is effected by performing centrifugation at 5,000—20,000 r.p.m. for 10—60 minutes to remove precipitates and recovering the supernatant.

In this treatment, too, a chloride ion-containing dissociable salt may be added as added to the buffer solution mentioned above, as desired. The preferred salt and addition level are as mentioned above.

(b—4) Dialysis:

The recovered fraction may then be dialyzed as desired.

For the dialysis, a 0.01—0.2 M buffer solution having a pH of 6—8 (for example, Tris buffer, etc.) is preferably used. The dialysis period is preferably not less than 10 hours. As for the addition of a chloride ion-releasing salt, the above applies.

(b—5) High-level purification:

Each heterologous protein is purified, as desired, to a higher level by a fractionation means suited for the heterologous protein to be purified (suitably by affinity chromatography using a monoclonal antibody).

Dosage form Preparation:

The highly purified heterologous protein is treated by a *per se* known technique suited for the purpose of the dosage form preparation. For use as a drug, the protein is subjected to treatment for removel of microorganisms and sterilization, as desired, and then made up into a liquid or dry preparation based on the results of investigations on the excipient, stabilizer and solvent.

The production method according to the invention can be applied to any heterologous protein which can be produced by a genetically engineered transformant. Interferon (α, β or γ), urokinase, HBsAg and TPA are examples of such protein.

BRIEF DESCRIPTION OF THE DRAWING

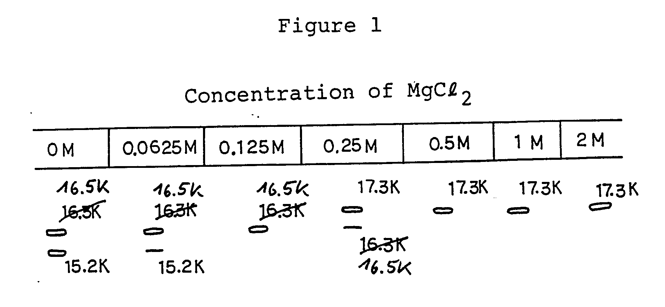

Fig. 1 shows the results of SDS-polyacrylamide gel electrophoresis of IFN-γ recovered in Experimental Example 1. After western blotting, IFN-γ was detected by staining by the enzyme antibody method using an anti-IFN-γ monoclonal antibody. In the figure, 16.6 K, 15.2 K and 17.3 K indicate the electrophoresed fractions having molecular weights of 16,600, 15,200 and 17,300, respectively.

EXAMPLES

The following working examples and experimental examples are further illustrative of the invention.

Example 1

The *Escherichia coli* strain W3110 carrying the plasmid PYS61 (Japanese Patent Application No. 59—229864), in which a DNA fragment coding for a human interferon-γ polypeptide is inserted, is cultivated overnight at 37°C in LB + glucose medium [prepared by dissolving 0.63 g of Tris base, 10 g of polypeptone, 5 g of yeast extract, 5 g of NaCl, 1 g of glucose and 20 mg of ampicillin in water and making the total volume 1 liter], and a 200-ml portion of this culture fliud is inoculated into 10 liters of KM—9 medium (prepared by dissolving 120 g of $Na_2HPO_4$, 60 g of $NaH_2PO_4$, 20 g of NaCl, 300 g of casamino acids and 50 g of yeast extract in water and making the total volume 10 liters). After 1 hour of cultivation at 37°C, indoleacrylic acid, which is a tryptophan gene inducer, is added to a concentration of 40 µg/ml and the cultivation is continued for further 7—10 hours. Cells are harvested by centrifuging the culture fluid at 4,000 r.p.m. for 20 minutes and washed with 30 mM NaCl + 50 mM Tris hydrochloride buffer (pH 8.0). The cells so washed are suspended in a lyzing buffer [30 mM NaCl, 50 mM EDTA, 260 mM Tris hydrochloride (pH 8.0), 10 µg/ml PMSF (phenylmethylsulfonyl fluoride), 0.5 M $MgCl_2$] to a cell concentration of $OD_{650} = 160$, and the suspension is charged into a Dyno-mill together with Ø 0.1 mm gliding beads. The Dyno-mill treatment is conducted at a revolution rate of 3,000 r.p.m. for 1—3 minutes.

Then, centrifugation is performed at 18,000 r.p.m. for 30 minutes to give a supernatant. This is dialyzed against 0.1 M Tris hydrochloride (pH 7.0) containing 0.5 M $MgCl_2$ and then assayed for interferon-γ (IFN-γ) by the cpe inhibition method [Havell, E. A. and Vilcek, J., Antimicrob. Agents Chemother., 2, 476—484 (1972)] [with Sindbis virus used as virus and human amnion-derived FL3—1 cells as animal cells]. The result thus obtained was $1.2 \times 10^9$ IU/liter.

The thus-obtained IFN-γ had a molecular weight of 17,300. No molecular weight reduction had been taken place during this treatment step.

Experimental Example 1

Following the procedure of Example 1, IFN-γ was extracted from the transformant and recovered for comparative investigation of the effect of salt addition ($MgCl_2$). The results are shown in Table 1. The salt was added to each of the buffer solution and the dialyzing solution. All the other conditions were the same as in Example 1. The data on the additive, addition level and IFN-γ activity are given in Table 1, while the data on the molecular weight of IFN-γ are given in Fig. 1. In the control run, the addition of $MgCl_2$ was omitted.

TABLE 1

| Additive | Final concentration | IFN-γ (IU/liter) |
|---|---|---|
| Control | —— | $3.9 \times 10^8$ |
| $MgCl_2$ | 0.125 | $6.1 \times 10^8$ |
| | 0.25 | $1.4 \times 10^9$ |
| | 0.5 | $1.5 \times 10^9$ |
| | 2 | $1.3 \times 10^9$ |
| | 5 | $1.0 \times 10^9$ |

Thus, the invention has succeeded in increasing the yield (about four times increased as compared with the conventional extraction and recovery methods) and preventing molecular weight reduction. As a result, the invention makes it possible to recover heterologous proteins at higher industrial levels even in the field of recombinant DNA technology, which uses *Escherichia coli* etc. as hosts and therefore has failed so far to achieve satisfactory production efficiency because of the property of intracellular accumulation.

**Claims**

1. A method of producing heterologous proteins, by cultivating genetically engineered, heterologous protein producing transformants to thereby cause production of said heterologous proteins, and of recovering the heterologous proteins thus produced, which comprises carrying out the treatment for recovery of said heterologous proteins from said transformants bearing the heterologous proteins produced therein, in the presence of magnesium chloride.

2. A method of producing heterologous proteins as claimed in Claim 1, wherein the concentration of the magnesium chloride is not less than 0.1 M.

3. A method of producing heterologous proteins as claimed in Claim 1, wherein the concentration of the magnesium chloride is 0.2—3 M.

**Patentansprüche**

1. Verfahren zur Herstellung heterologer Proteine durch Kultivierung gentechnisch veränderter, heterologes protein produzierender Transformanden zur Produktion dieser heterologen Proteine und zur Wiedergewinnung der auf diese Weise produzierten heterologen Proteine, wobei die Behandlung zur Wiedergewinnung dieser heterologen Proteine von den genannten Transformanden, die die in ihnen produzierten heterologen Proteine enthalten, in Gegenwart von Magnesiumchlorid vorgenommen wird.

2. Verfahren zur Herstellung heterologer Proteine nach Anspruch 1, worin die Konzentration des Magnesiumchlorids nicht weniger als 0,1 M beträgt.

3. Verfahren zur Herstellung heterologer Proteine nach Anspruch 1, worin die Konzentration des Magnesiumchlorids im Bereich von 0,2 bis 3 M liegt.

**Revendications**

1. Procédé de production de protéines hétérologues consistant à cultiver des transformants produisant des protéines hétérologues formés par génie génétique pour ainsi provoquer la production desdites protéines hétérologues et à récupérer les protéines hétérologues ainsi produites, procédé qui consiste à effectuer le traitement de récupération desdites protéines hétérologues à partir desdits transformants portant les protéines hétérologues produites dans eux, en présence de chlorure de magnésium.

2. Procédé de production de protéines hétérologues selon la revendication 1, dans lequel la concentration du chlorure de magnésium n'est pas inférieure à 0,1 M.

3. Procédé de production de protéines hétérologues selon la revendication 1 dans lequel la concentration du chlorure de magnésium est de 0,2 à 3 M.

## Figure 1

### Concentration of $MgCl_2$

| 0 M | 0.0625M | 0.125M | 0.25 M | 0.5M | 1 M | 2 M |
|---|---|---|---|---|---|---|

16.5K    16.5k    16.5k    17.3K    17.3K    17.3K    17.3K

16.3K    16.3K    16.3K

15.2K    15.2K      16.3K

16.5k